# EUROPEAN PATENT SPECIFICATION

(11) **EP 3 311 809 B1**
(45) Date of publication and mention of the grant of the patent: **13.05.2020**
(21) Application number: 15895255.6
(22) Date of filing: 18.06.2015
(51) Int. Cl.: A61K 31/366, A61K 31/728, A61K 38/39, A61K 9/16, A61P 19/02, A61P 19/00

(54) **USE OF PHARMACEUTICAL COMPOSITION IN PREPARATION OF DRUG FOR PROMOTING CHONDROCYTE GENERATION**
VERWENDUNG EINER PHARMAZEUTISCHEN ZUSAMMENSETZUNG BEI DER HERSTELLUNG EINES ARZNEIMITTELS ZUR FÖRDERUNG DER CHONDROZYTENPRODUKTION
UTILISATION D'UNE COMPOSITION PHARMACEUTIQUE DANS LA PRÉPARATION D'UN MÉDICAMENT POUR FAVORISER LA GÉNÉRATION DE CHONDROCYTES

(43) Date of publication of application: 25.04.2018
(73) Proprietor: Kaohsiung Medical University, Kaohsiung 807 (TW)
(72) Inventor: HO, Mei-Ling, Kaohsiung 807 (TW); CHANG, Je-Ken, Kaohsiung 807 (TW); WU, Shun-Cheng, Kaohsiung City 806 (TW); CHANG, Chih-Hsiang, Taichung City 407 (TW)
(74) Representative: Ter Meer Steinmeister & Partner
(86) International application number: PCT/CN2015/081835
(87) International publication number: WO 2016/201682

(56) References cited:
- WO-A2-2009/092094
- CN-A- 103 705 505
- J. KRAMER ET AL: "Simvastatin modulates mouse embryonic stem cell-derived chondrogenesis in vitro", TOXICOLOGY IN VITRO., vol. 26, no. 7, 1 October 2012 (2012-10-01), pages 1170-1176, XP55347917, GB ISSN: 0887-2333, DOI: 10.1016/j.tiv.2012.06.013
- HUINA ZHANG ET AL: "Simvastatin Stimulates Chondrogenic Phenotype of Intervertebral Disc Cells Partially Through BMP-2 Pathway :", SPINE : AN INTERNATIONAL JOURNAL FOR THE STUDY OF THE SPINE, vol. 33, no. 16, 1 July 2008 (2008-07-01), pages E525-E531, XP55526307, US ISSN: 0362-2436, DOI: 10.1097/BRS.0b013e31817c561b

## Description

### TECHNICAL FIELD

The present invention relates to a pharmaceutical composition for use in promoting chondrogenesis, specifically, a pharmaceutical composition comprising hyaluronic acid and simvastatin for promoting chondrogenesis.

### BACKGROUND

Cartilage tissue is a connective tissue lacking blood vessels, lymphatic system and nerves, composed primarily of hyaline cartilage, and hyaline cartilage is composed primarily of chondrocytes, type II collagen, and proteoglycan. Once the cartilage tissue is damaged, the number of chondrocytes located in the immediate vicinity is too limited to repair the damage, moreover, it is too difficult for chondrocytes to migrate to the damaged site because they are coated by extracellular matrix. It is currently known that most of the new tissues generated by cartilage self-repair is fibrocartilage tissue which is primarily type I collagen. Fibrocartilage will gradually degrade because it lacks the biomechanics of cartilage and the function of hyaline cartilage, which makes it difficult for joints to regain normal pre-injury conditions for movement. Applications of tissue engineering to cartilage tissue repair have been rapidly developing in recent years. In this approach, chondrocytes or mesenchymal stem cells are used to generate viable and functional articular cartilage tissues. The use of mesenchymal stem cells as a cell source is currently acknowledged as an approach of high potential. Although mesenchymal stem cells are capable of being differentiated into chondrocytes, if they are to be used to repair articular cartilage tissues, inducible factors are needed to induce chondrogenic differentiation in mesenchymal stem cells. However, inducible factors of protein origins are expensive and involve with problems such as protein denaturation or pathogen contamination. When chemical inducible factors are used, other side effects are involved. It has been reported in the past that bone morphogenetic protein-2 (BMP-2) is capable of inducing chondrogenesis in stem cells and inducing ossification in osteoblasts, therefore can be used in articular cartilage and bone tissue engineering. However, there is a concern about the risk of osteosclerosis when articular cartilage is repaired by inducing the expression of BMP-2. Previous studies have already reported that though cartilage defects can be repaired by inducing high expression of BMP-2 in stem cells, bone spurs may be formed at the repaired sites (Arthritis Rheum. Articular cartilage repair by gene therapy using growth factor-producing mesenchymal cells. 2003 Feb; 48 (2): 430-41.) How to stimulate stem cells to express large amounts of BMP-2 while avoiding ossification caused by BMP-2 is still an unsolved problem.

Statin drugs, including simvastatin, are mainly used clinically to lower blood lipids. Moreover, they can induce expression of BMP-2 in chondrocytes, production of chondrogenic genes (type II collagen and aggrecan) and extracellular matrix. Statin drugs also can induce expression of BMP-2 in osteoblasts to repair bone fractures by enhancing ossification. Therefore, though statin drugs can be used to induce BMP-2 expression for cartilage repair, how to induce high expression of BMP-2 in stem cells to repair cartilage defects while avoiding ossification caused by BMP-2 is yet an unsolved problem.

### DETAILED DESCRIPTION OF THE INVENTION

The present invention has demonstrated by experiments that when adipose-derived stem cells are treated with a hyaluronic acid-containing microenvironment and simvastatin, not only the production of BMP-2, chondrogenic genes (Sox-9, aggrecan and collagen Type II), and articular cartilage extracellular matrix (sGAG) can be enhanced, the expression of osteocalcin induced by simvastatin can be reduced at the same time to suppress osteosclerosis, which can be used to repair cartilage defects.

The present invention discovers that simvastatin together with hyaluronic acid have the effect of inducing chondrogenesis in stem cells. In addition, the expression of BMP-2 is more effectively enhanced, the expression of chondrogenic genes is increased, and the level of sulfated glycosaminoglycan (a major extracellular matrix in articular cartilage) produced by adipose-derived stem cells is increased when a three-dimensional hydrogel carrier is further formed from fibrin to coat hyaluronic acid and stem cells, and a simvastatin-containing microsphere is also added into the carrier. In addition, it is found that the present composition (combining adipose-derived stem cells with simvastatin and hyaluronic acid) effectively repairs articular cartilage defects in a miniature pig cartilage defect model.

As used herein, the term "a" or "an" are employed to describe elements and components of the present invention. This is done merely for convenience and to give a general sense of the present invention. This description should be read to include one or at least one and the singular also includes the plural unless it is obvious that it is meant otherwise.

The present invention provides a pharmaceutical composition comprising a hyaluronic acid-containing mixture and a statin drug, a dose kit, and a therapeutic method.

The present invention provides a pharmaceutical composition for use in promoting chondrogenesis, wherein the pharmaceutical composition comprises a hyaluronan-containing mixture and a statin compound.

As used herein, the term "promoting chondrogenesis" includes, but is not limited to, promoting cells to synthesize, metabolize, express or secrete factors or hormones associated with chondrogenic differentiation so that the cells form chondrocytes.

In one embodiment, the hyaluronic acid-containing mixture comprises hyaluronic acid, fibrin and a stem cell. In a preferred embodiment, the fibrin forms a hydrogel. The fibrin form a scaffold structure of a three-dimensional hydrogel, i.e., a 3D fibrin hydrogel, which is used as a carrier to coat the hyaluronic acid and the stem cell.

As used herein, the term "hydrogel" refers to a gel using water as a dispersion medium, constituted by introducing a partially hydrophobic group and hydrophilic residues into a water-soluble polymer having a network-like cross-linked structure to form a cross-linked polymer, wherein water molecules are connected within the network, and the hydrophobic residues are water-swollen. Therefore, any water-soluble or hydrophilic polymers can from hydrogels through certain chemical or physical crosslink. Hydrogels can be categorized into physical hydrogels and chemical hydrogels: (1) physical hydrogels are formed by physical forces such as electrostatic interactions, hydrogen bonding, chain entanglement, etc. Such gels are non-permanent, can be transformed into solution under heat, they are also known as pseudogels or thermoreversible gels. Many natural polymers are in a state of stable hydrogels at room temperature, such as kappa-2 carrageenan, agar; as for synthetic polymers, polyvinyl alcohol (PVA) is a typical example, a hydrogel which is stable below 60°C and can be obtained after a freezing and thawing treatment. (2) Chemical hydrogels are three-dimensional network polymers crosslinked by chemical bonds, they are permanent and also known as true gels. There are macroscopic hydrogels and microscopic hydrogels (microspheres) which are categorized according to the shape and the size of each hydrogel. According to the shape, macroscopic hydrogels can be further categorized into columnar, porous sponge-like, fibrous, film-like, spherical hydrogels. Currently, prepared microspheres are categorized into micron-sized hydrogels and nano-sized hydrogels.

As used herein, the term "stem cell" refers to a group of cells that are capable of self-renewal and proliferation while maintaining an undifferentiated state over a long period of time. In addition, this group of cells have multi-differentiation potential, capable of differentiating into cell populations of different lineages and functionally specific tissues after being properly induced and stimulated. The term "human mesenchymal stem cells" refers to any cells which are derived from human stroma tissue and capable of unlimited self-renewal and differentiating into a variety of cells or tissue types, such as, but not limited to, one selected from the group consisting of adipose-derived mesenchymal stem cells, bone marrow-derived mesenchymal stem cells, umbilical cord-derived mesenchymal stem cells, periosteum-derived mesenchymal stem cells, synovium-derived mesenchymal stem cells, and muscle-derived mesenchymal stem cells. The examples of the present invention are illustrated by the examples of human adipose-derived mesenchymal stem cells (or known as human adipose-derived stem cells, hADSCs). However, the present invention is not limited by the following examples. In one embodiment, the stem cells comprise a human dermal fibroblast, a mesenchymal stem cell or an adipose-derived stem cell. In a preferred embodiment, the stem cells comprise human adipose-derived stem cells (hADSCs).

In one embodiment, the concentration of the hyaluronic acid ranges from 0.1 wt% to 10 wt%. In a preferred embodiment, the concentration of the hyaluronic acid ranges from 0.5 wt% to 5 wt%. In a more preferred embodiment, the concentration of the hyaluronic acid is 1 wt%.

As used herein, the term "statin compound" includes, but is not limited to, atorvastatin, cerivastatin, fluvastatin, lovastatin, mevastatin, pitavastatin, pravastatin, rosuvastatin or simvastatin. In a preferred embodiment, the statin compound is simvastatin. The structural of simvastatin is as following formula (I):

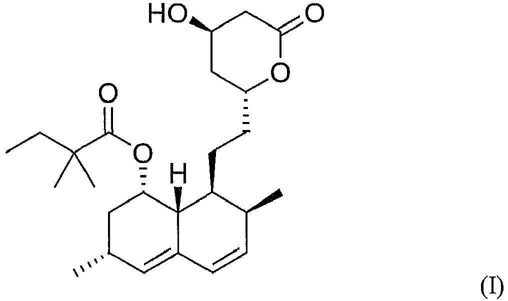

In one embodiment, the concentration of simvastatin ranges from 0.1 µM to 10 µM. In a preferred embodiment, the concentration of simvastatin ranges from 0.5 µM to 5 µM. In a more preferred embodiment, the concentration of simvastatin is 1 µM. In another embodiment, the simvastatin is in the dosage form of a microsphere. The microsphere dosage form can effectively control the concentration of the released simvastatin.

Thus, the pharmaceutical composition of the present invention can be administered to a subject as a composition comprising a fibrillar hydrogel having hyaluronic acid and stem cells, and a simvastatin-containing microsphere to promote chondrogenesis or to repair cartilage defects. In addition, the simvastatin-containing microsphere can be administered to the subject by being directly added into the fibrillar hydrogel having hyaluronic acid and stem cells.

As used herein, the term "cartilage defects" includes, but is not limited to, articular cartilage degeneration or diseases of articular cartilage defects/wear caused by gene mutations or damages cause by external force. The articular cartilage widely exists in the articular surface of a bone, costal cartilage, trachea, pinna, lumbar discs.

It is currently known that many factors or triggering agents stimulate anabolic metabolism of chondrocytes, for example, insulin-like growth factor-I (IGF-I) is the principal growth factor for anabolic metabolism in synovial fluid, it also stimulates the synthesis of proteoglycans and collagens; in addition, members of the bone morphogenic protein (BMP) family (in particular BMP2, BMP4, BMP6 and BMP7) and members of the human transforming growth factor-β (TGF-β) family can induce the anabolic metabolism of chondrocytes (Chubinskaya and Kuettner, 2003). Further, a compound which can induce the anabolic metabolism of chondrocytes has been identified in recent years (US 6,500,854; EP 1 391 211). Therefore, in a preferred embodiment, the effect of promoting chondrogenesis of the present invention is to induce chondrification in cells or stem cells by increasing or enhancing and stimulating factors or hormones associated with the anabolic metabolism of chondrocytes. In a more preferred embodiment, the promoting chondrogenesis is achieved by increasing the expression of bone morphogenetic protein-2 (BMP-2).

Further, two major components of the extracellular matrix of the articular cartilage are proteoglycan and type II collagen (Col II). Glycosaminoglycan (GAG) is the glycan moiety in proteoglycan, the structure of which is characterized by having sulfated disaccharide repeating units. Type II collagen and proteoglycans form the framework of cartilage, providing the cartilage with stability and elasticity. In addition to being the structural framework of tissues, the extracellular matrix (ECM) modulates chondrocytes through cellular signaling mechanisms. These complex interactions affect gene expression in chondrocytes at the mRNA and protein expression level, altering the intracellular state of the cells. In addition, the degradation of extracellular matrix is often associated with the pathological state of the cartilage, in which the expression level of type II collagen is reduced and the viability of matrix metalloproteinases (MMPs) is enhanced. Further, growth factors such as TGF-β1 and IGF-I are involved in the secretion and maintenance of the extracellular matrix of articular chondrocytes. Therefore, in a preferred embodiment, the efficacy of the present invention for promoting chondrogenesis is achieved by increasing the production of extracellular matrix and stimulating the expression of factors associated with the maintenance/synthesis of the extracellular matrix, thereby inducing chondrogenesis of cells or stem cells to form cartilage. In a more preferred embodiment, the promotion of chondrogenesis is achieved by increasing the production of sulfated glycosaminoglycans (sGAG).

Chondrogenic genes can induce chondrogenesis of cells, chondrogenic genes include, but are not limited to, Sox-9, aggrecan or type II collagen. In one embodiment, the promotion of chondrogenesis is achieved by enhancing the expression of chondrogenic genes, wherein the chondrogenic genes comprise Sox-9, aggrecan or type II collagen.

As used herein, the term "expression" includes, but is not limited to, the expression of genes, RNAs, or proteins.

In addition, most compounds that stimulate the anabolic metabolism of chondrocytes have serious side effects, for example, overexpression of BMP-2 leading to the formation of bone spurs, i.e., occurrence of osteosclerosis. Therefore, currently there is no compound that can stimulate chondrogenic differentiation without such side effects. Osteogenic factors or factors inducing osteosclerosis include, but are not limited to, osteocalcin. Therefore, the present invention not only promotes chondrogenesis by strengthening various factors associated with chondrogenesis, but also avoids the occurrence of osteosclerosis while chondrocytes are produced. In one embodiment, the pharmaceutical composition further inhibits osteosclerosis of the produced chondrocytes. In a preferred embodiment, the inhibition of osteosclerosis of the produced chondrocytes is achieved by inhibiting the expression of osteocalcin.

The present invention also relates to a pharmaceutical composition for use in treating cartilage defects by administering a therapeutically effective amount to a cartilage defect site of a subject, wherein the pharmaceutical composition comprises a hyaluronic acid (HA) mixture and a statin compound. In one embodiment, the hyaluronic acid mixture comprises hyaluronic acid, fibrin, and a stem cell. In a preferred embodiment, the fibrin forms a hydrogel. The fibrin forms the scaffold structure of a three-dimensional hydrogel, that is a 3D fibrin hydrogen, which is used as a carrier for coating the hyaluronic acid and the stem cell.

In another embodiment, the statin compound is simvastatin. The statin compound of the present invention, such as simvastatin, may be embedded in, for example, microcapsules prepared by coacervation techniques or by interfacial polymerization (e.g. carboxymethyl cellulose or gelatin microcapsules, respectively, and poly-(methyl methacrylate) microcapsules), colloidal drug delivery systems (for example, liposomes, microspheres, microemulsions, nanoparticles or nanocapsules), or macroemulsions. In a preferred embodiment, the simvastatin is in a dosage form of a microsphere. The microsphere can effectively control the concentration of the released simvastatin.

As used herein, the term "therapeutically effective amount" refers to an amount of a compound, when administered to a subject to treat a disease, that is sufficient to effect the desired treatment of the disease. The "therapeutically effective amount" may vary depending on the compound, the disease and its severity, and the age, weight, etc. of the subject to be treated. In one embodiment, the therapeutically effective amount of the hyaluronic acid ranges from 0.1 to 10 wt%. In a preferred embodiment, the therapeutically effective amount of the hyaluronic acid ranges from 0.5 to 5 wt%. In a more preferred embodiment, the therapeutically effective amount of the hyaluronic acid is 1 wt%. In another embodiment, the therapeutically effective amount of the simvastatin ranges from 0.1 to 10 µM. In a preferred embodiment, the therapeutically effective amount of the simvastatin ranges from 0.5 to 5 µM. In a more preferred embodiment, the therapeutically effective amount of the simvastatin is 1 µM.

The term "treat" refers to any improvements of a disease or illness (also refers to inhibition of the disease or amelioration of the appearance, extent or severity of at least one of its clinical symptoms).

As used herein, the term "subject" refers to an animal. In a preferred embodiment, the subject refers to a mammal. In a more preferred embodiment, the subject refers to a human.

The pharmaceutical compositions of the present invention may be prepared in a manner well known in the art of pharmacy. In general, a pharmaceutically effective amount of the pharmaceutical composition of the present invention is administered. The dose of the pharmaceutical composition actually administered is generally determined by physicians in accordance with the following relevant conditions, including: the illness to be treated; the selected route of administration; the compound actually administered; the age, weight and response of each individual patient; the severity of the patient's symptoms; and the likes.

The preferred route of administration of the pharmaceutical composition of the present invention to the subject is intraarticular administration. The pharmaceutical compositions of the present invention may also be administered in a sustained release dosage form or administered through a sustained release drug delivery system.

The present disclosure further provides a kit, which comprises a hyaluronic acid mixture and a statin compound.

In one embodiment, the hyaluronic acid mixture comprises hyaluronic acid (HA), fibrin, and a stem cell. In a preferred embodiment, the fibrin forms a hydrogel. The fibrin forms the scaffold structure of a three-dimensional fibrin hydrogel, that is a 3D fibrin hydrogel, which is used as a carrier for coating the hyaluronic acid and the stem cell.

In another embodiment, the statin compound is simvastatin. In a preferred embodiment, the simvastatin is in the dosage form of a microsphere.

In one embodiment, the fibrin hydrogel which contains hyaluronic acid and stem cells, and the simvastatin-containing microsphere may be in separate dosage forms, but administered together when used. Alternatively, the simvastatin-containing microsphere may be administered in a single dosage form after being dissolved in the fibrin hydrogel.

When compared with other commonly used technologies, the pharmaceutical composition provided by the present invention further has the following advantages:

(1) When hyaluronic acid is used in combination with a statin compound, factors associated with chondrogenesis or factors inducing chondrification of cells, such as bone morphogenetic protein (BMP), cartilage extracellular matrix (such as sGAG or Col II), chondrogenic genes (such as Sox-9, aggrecan or type II collagen), are significantly and effectively potentiated , and thus can be used for the treatment of various diseases associated with cartilage defects/wear.

(2) Currently, many commercially available medications or reagents for treating cartilage defects or for stimulating cartilage growth have the potential of causing osteosclerosis, which is probably caused by over-expression of chondrogenic factors. However, the pharmaceutical composition of the present invention not only significantly increases cartilage formation or induces the expression of factors associated with chondrogenesis in cells, but further inhibits the expression of osteosclerosis factors, for example, reduces the expression level of osteocalcin, to prevent the occurrence of osteosclerosis.

### BRIEF DESCRIPTION OF THE DRAWINGS

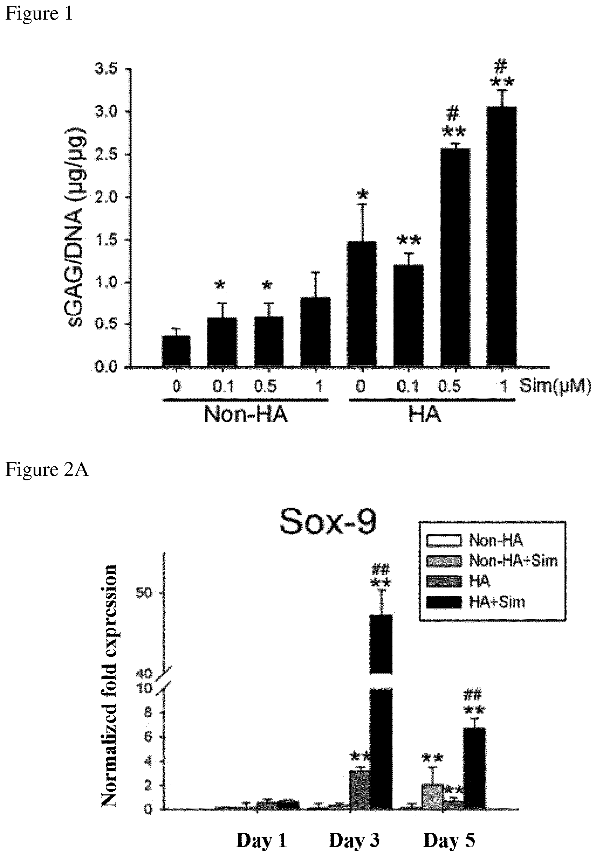

Figure 1 shows the effect of simvastatin on the expression of sulfated glycosaminoglycans (sGAG) in human adipose-derived stem cells (hADSCs) cultured in wells coated with hyaluronic acid (HA) or without hyaluronic acid (* and **: as compared to the group not treated with hyaluronic acid and simvastatin, * p <0.05; ** p <0.01. #: as compared to the group treated with simvastatin (0 µM), P <0.01. The number of experiments is 3).
Figure 2A shows the effect of simvastatin on the expression of chondrogenic gene Sox-9 in human adipose-derived stem cells cultured with hyaluronic acid (HA) or without hyaluronic acid (non-HA) (as compared to the group with no hyaluronic acid (non-HA), * * p <0.01. The number of experiments is 4).

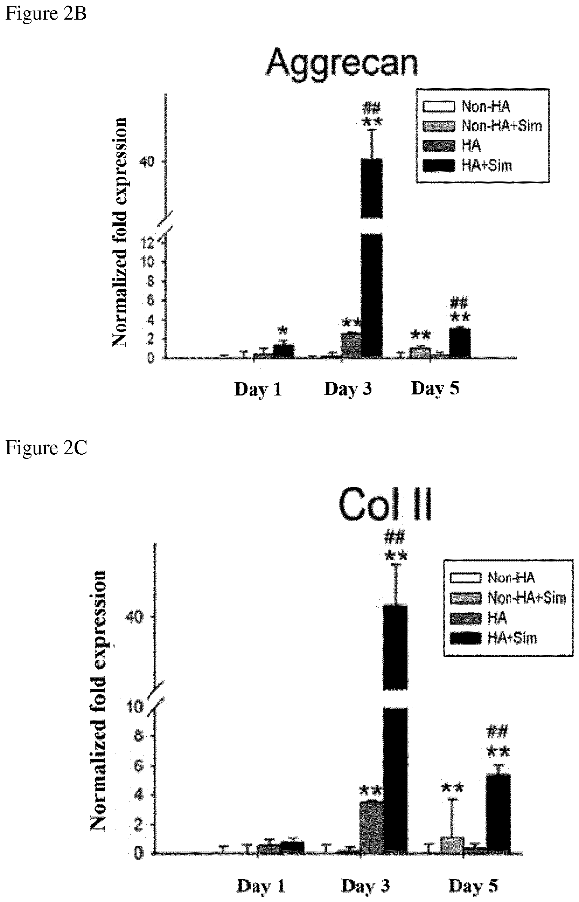

Figure 2B shows the effect of simvastatin on the expression of chondrogenic gene aggrecan in human adipose-derived stem cells cultured with hyaluronic acid (HA) or without hyaluronic acid (Non-HA) (as compared to the group with no hyaluronic acid (non-HA), ** p <0.01. The number of experiments is 4).
Figure 2C shows the effect of simvastatin on the expression of chondrogenic gene Col II in human adipose-derived stem cells cultured with hyaluronic acid (HA) or without hyaluronic acid (Non-HA) (as compared to the group with no hyaluronic acid (non-HA), ** p <0.01. The number of experiments is 4).

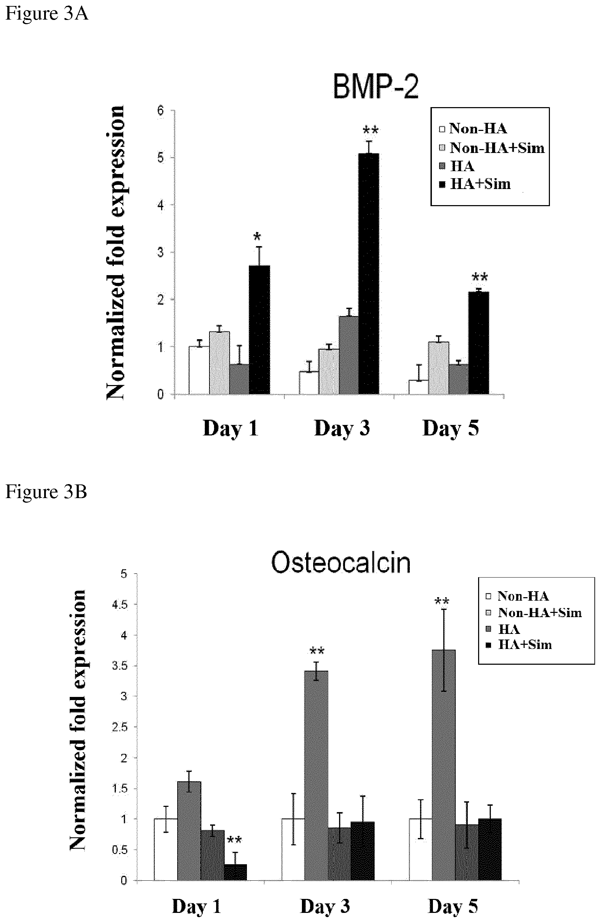

Figure 3A shows the effect of simvastatin on gene expression of BMP-2 in human adipose-derived stem cells cultured with hyaluronic acid (HA) or without hyaluronic acid (Non-HA) (as compared to the data of the first day of the group with no hyaluronic acid (non-HA), ** p <0.01. The number of experiments is 4).
Figure 3B shows the effect of simvastatin on gene expression of osteocalcin in human adipose-derived stem cells cultured with hyaluronic acid (HA) or without hyaluronic acid (Non-HA) (as compared to the data of the first day of the group with no hyaluronic acid (non-HA), ** p <0.01. The number of experiments is 4).

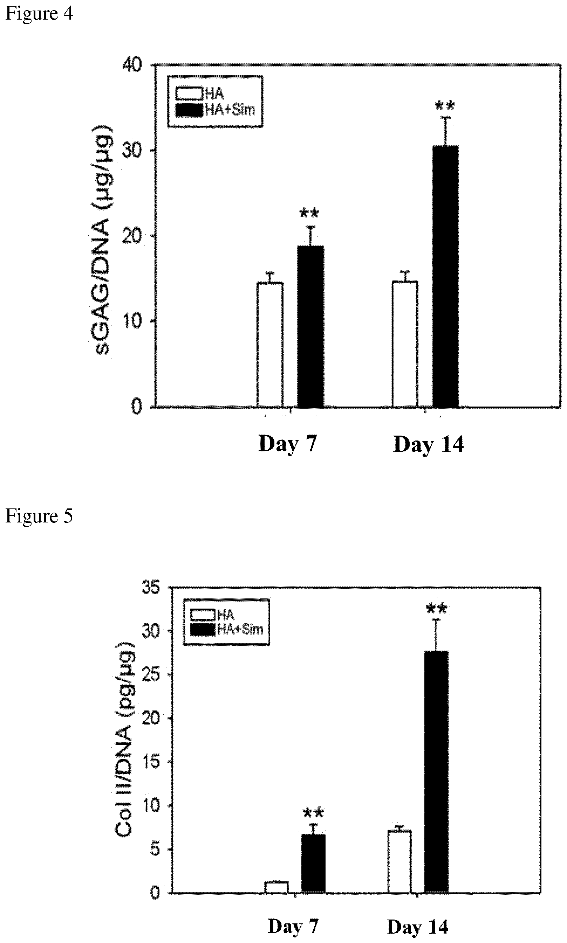

Figure 4 is the result analysis of the formation and expression of sulfated glycosaminoglycans (sGAG) in human adipose-derived stem cells coated with a three-dimensional hyaluronic acid/fibrin hydrogel and treated with simvastatin (as compared to the hyaluronic acid group (HA) and hyaluronic acid plus Simvastatin group (HA+Sim) on the same days, ** p <0.01. The number of experiments is 3).
Figure 5 is the result analysis of the expression of type II collagen (Col II) in human adipose-derived stem cells coated with a three-dimensional hyaluronic acid/fibrin hydrogel and treated with simvastatin (as compared to the hyaluronic acid (HA) group and hyaluronic acid plus Simvastatin (HA+Sim) group on the same days, ** p <0.01. The number of experiments is 3).

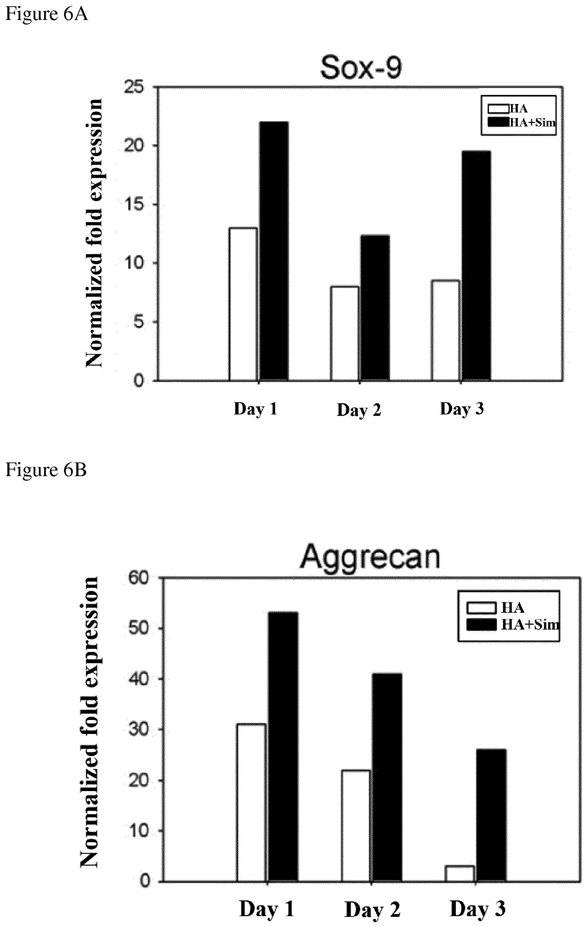

Figure 6A is the result analysis of the expression of chondrogenic gene Sox-9 in human adipose-derived stem cells coated with a three-dimensional hyaluronic acid/fibrin hydrogel and treated with simvastatin.
Figure 6B is the result analysis of the expression of chondrogenic gene aggrecan in human adipose-derived stem cells coated with a three-dimensional hyaluronic acid/fibrin hydrogel and treated with simvastatin.

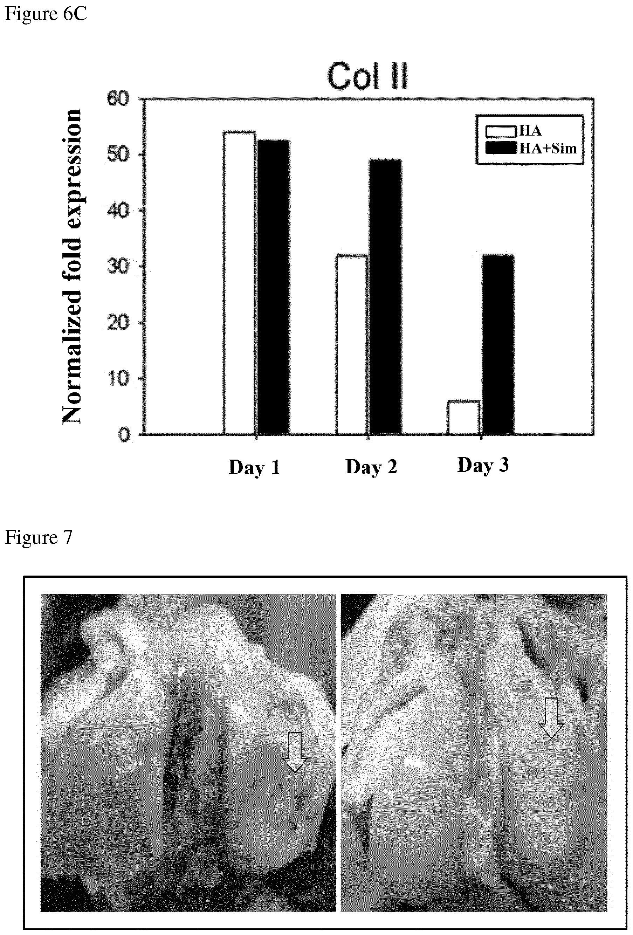

Figure 6C is the result analysis of the expression of chondrogenic gene Col II by human adipose-derived stem cells coated with a three-dimensional hyaluronic acid/fibrin hydrogel and treated with simvastatin.
Figure 7 shows the results of the articular cartilage defects in the knee joints of miniature pigs treated with the three-dimensional hyaluronic acid/fibrin hydrogel containing simvastatin and human adipose-derived stem cells (arrows show where the knee joint are repaired).

### EXAMPLES

The present invention may be embodied in a variety of contents and is not limited to the examples below. The examples below are merely representative of various aspects and features of the present invention.

### Example 1

Sulfated glycosaminoglycan (sGAG) is a major extracellular matrix of articular cartilage. In order to test whether simvastatin can promote the expression of sGAG in human adipose-derived stem cells (hADSCs) and its optimal concentration, the present invention first prepared hyaluronic acid (HA) coated wells by coating hyaluronic acid dissolved in 1 ml of phosphate buffer (PBS, 1% w/w) on a 24-well plate, standing at 37 °C for 48 hours, then washed with PBS two times. Human adipose-derived stem cells were seeded in wells containing 500 µl of basal medium at a density of 1×10⁵ cells per well, wherein the basal medium contains Dulbecco's modified Eagle's medium (DMEM), and was additionally supplemented with 5% fetal bovine serum (FBS), 1% nonessential amino acids and 100 U/ml of penicillin/streptomycin (Gibco-BRL, Grand Island, NY). During the culturing period, simvastatin at different concentrations (0.1 to 1 µM) was added to the well plate, and the medium was replaced with fresh one every two days. On day 7 after the cultivation started, cells were collected for dimethylmethylene blue (DMMB) analysis with Blyscan sulfated glycosaminoglycan reagent (Biocolor, Antrim, UK), and for standardization of sulfated glycosaminoglycan (sGAG) formation with DNA. The results were shown in Figure 1.

The present invention tested the effect of simvastatin on the formation of sulfated glycosaminoglycans (sGAG) in human adipose-derived stem cells in the presence or absence of hyaluronic acid. As show in Figure 1, in the absence of hyaluronic acid (Non-HA), the expression levels of sGAG in human adipose-derived stem cells were all enhanced when treated with 0.1∼1 µM of simvastatin (0 µM: 0.3 µg/µg; 0.1 µM: 0.5 µg/µg; 0.5 µM: 0.5 µg/µg; 1 µM: 0.7 µg/µg); in the presence of 1% hyaluronic acid, the expression levels of sGAG were further enhanced when treated with 1 µM of simvastatin (0 µM: 1.4 µg/µg; 0.1 µM: 1.3µg/µg; 0.5 µM: 2.5 µg/µg; 1 µM: 2.8 µg/µg). This experiment showed that when 1 % hyaluronic acid and 1 µM of simvastatin worked together, the expression of sGAG in stem cells was significantly and preferably enhanced to induce chondrification of cells, thereby promoting cartilage formation.

### Example 2

Human adipose-derived stem cells (hADSCs) were cultured in wells coated with hyaluronic acid (HA) and without hyaluronic acid (non-HA), and treated with or without 1 µM of simvastatin (SIM) for 1 to 5 days. The present invention was therefore divided into four experimental groups: (1) No hyaluronic acid (Non-HA) group: human adipose-derived stem cells were cultured in wells not coated with hyaluronic acid and not treated with simvastatin (1 µM); (2) No hyaluronic acid plus simvastatin (Non-HA+Sim) group: human adipose-derived stem cells were cultured in wells not coated with hyaluronic acid but treated with simvastatin (1 µM); (3) Hyaluronic acid (HA) group: human adipose-derived stem cells were cultured in wells coated with hyaluronic acid but not treated with simvastatin (1 µM); and (4) Hyaluronic acid plus simvastatin (HA+Sim) group: human adipose-derived stem cells were cultured in wells coated with hyaluronic acid and treated with simvastatin (1 µM).

Cells were harvested on designated days and analyzed for the expression of BMP-2 and chondrogenic genes using real-time PCR. Total RNA of these cells was extracted by following the manufacturer's instructions and using TRIzol reagent (Gibco BRL, Rockville, MD). In short, a reaction volume of 20 µl was taken which contained 0.5 to 1 µg of total RNA, and then RNA was reverse-transcribed into cDNA by using the SuperScript First-Strand Synthesis System (Invitrogen). Real-time PCR reactions were performed and monitored by using iQ™ SYBR green® supermix (Bio-Rad Laboratories Inc, Hercules, CA) and a quantitative real-time PCR detection system (Bio-Rad Laboratories Inc, Hercules, CA). The cDNA sample (2 µl was taken to be used in a total volume of 25 µl for each reaction) was used for analysis of those genes of interest and the reference gene glyccraldehyde 3-phosphate dehydrogenase (GAPDH) was used as a control group. The expression level of each of the target genes, as previously described, was calculated as 2^{-ΔΔ}Ct. For each gene of interest, four readings were performed for each of the experimental samples, each experiment was repeated for at least three times. Applied primer sets were as follows: (1) bone morphogenetic protein-2 (BMP-2), forward: CGAATGACTGGATTGTGGCT, reverse: TGAGTTCTGTCGGGACACAG; (2) Sox-9, forward: CTT CCG CGA CGT GGA CAT, reverse: GTT GGG CGG CAG GTA CTG; (3) Collagen type II (Col II), forward: CAA CAC TGC CAA CGT CCA GAT, reverse: TCT TGC AGT GGT AGG TGA TGT TCT; (4) aggrecan, forward: ACA CGT GGG GAC AGT ATT GG, reverse: GTG GAA AGA TGC GGT GGT TT; (5) GAPDH, forward: TCT CCT CTG CAT TCA ACA GCGAC, reverse: CCC TGT TGT TGC AGC CAA ATT C; and (6) osteocalcin, forward: GTG CAG AGT CCA GCA AAG GT, reverse: CGA TAG GCC TCC TGA AAG C, the results are shown in Figure 2 and Figure 3.

The present invention first tested whether the composition of 1% of hyaluronic acid (HA) and 1 µM of simvastatin stimulated the expression of chondrogenic genes (such as Sox-9, aggrecan and Col II) and their preferred concentrations. As shown in Figures 2A to 2C, in the absence of hyaluronic acid (Non-HA), 1 µM of simvastatin failed to effectively enhance the expression of chondrogenic genes (Non-HA+ Sim); however, in the presence of hyaluronic acid, better expression levels of chondrogenic genes were achieved when 1 µM of simvastatin (HA+Sim) was administered, that is, increased expression levels of Sox-9, aggrecan and Col II. This experiment showed that when 1% of hyaluronic acid and 1 µM of simvastatin worked together, the expression of chondrogenic genes in stem cells was significantly and preferably enhanced.

The present invention tested whether the composition of 1% of hyaluronic acid and 1 µM of simvastatin stimulated the expression of BMP-2 and osteocalcin in the presence or in the absence of hyaluronic acid. As shown in Figure 3A, when the expression level of BMP-2 on the first day of the non-hyaluronic acid group (Non-HA) was regarded as 1 for the purpose of comparison, in the absence of hyaluronic acid (Non-HA) the expression of BMP-2 gene was not significantly enhanced by giving 1 µM of simvastatin (day 1: Non-HA group: 1 fold, Non-HA+Sim group: 1.2 fold; day 3: Non-HA group: 0.5 fold, Non-HA+Sim group: 0.8 fold; day 5: Non-HA group: 0.2 fold, Non-HA+Sim group: 1.1 fold.) In the presence of 1% of hyaluronic acid and 1 µM of simvastatin was given (HA+Sim), the expression of BMP-2 was significantly increased (day 1: HA: 0.5 fold, HA+Sim: 2.8 fold; day 3: HA: 1.9 fold, HA+Sim: 5 fold; day 5: HA: 0.4 fold, Non-HA+Sim: 2.1 fold), this experiment showed that when 1% of hyaluronic acid and 1 µM of simvastatin worked together, the expression of BMP-2 gene was significantly increased.

In terms of gene expression level of osteocalcin, in Figure 3B, when the expression level of osteocalcin on the first day of the Non-HA group was regarded as 1 for the purposes of comparison, 1 µM of simvastatin was given in the absence of hyaluronic acid (Non-HA), the expression of osteocalcin gene was significantly increased from day 1 to day 5, however, when 1 µM of simvastatin was given in the presence of HA (HA+Sim), the expression of osteocalcin induced by simvastatin was effectively reduced from day 1 to day 5. The above results indicated that when hyaluronic acid and simvastatin worked together the expression of osteocalcin was reduced, thereby decreasing the occurrence of osteosclerosis.

### Example 3

30 µL of human adipose-derived stem cells (5×10⁶ cells) suspended in 1% of hyaluronic acid was mixed with 120 µl of fibrin solution (100 mg/ml), and then placed in a teflon mold (5.5 mm in depth and 5.5 mm in diameter). 40 µl of bovine thrombin (300 U/ml) dissolved in 40 mM of CaCl₂ was added into the mold and mixed well with the cell/fibrin solution. The mixture was incubated for 15 minutes at room temperature until a hydrogel was formed. After the hydrogel was formed, the three-dimensional fibrin hydrogel carrier containing human adipose-derived stem cells and hyaluronic acid (hyaluronic acid/fibrin hydrogel) was removed from the Teflon mold, and then moved to a 24-well plate, cultivated with 1 ml of basal medium (DMEM), wherein the basal medium contained Dulbecco's modified Eagle's medium (DMEM), and was additionally supplemented with 5% of fetal bovine serum (FBS), 1% of nonessential amino acids and 100 U/ml of penicillin/streptomycin (Gibco-BRL, Grand Island, NY). The human adipose-derived stem cells (hADSCs) were cultured in the hyaluronic acid/fibrin hydrogel and treated with or without 1 µM of simvastatin for 7 or 14 days. Therefore, there were two experimental groups in the present invention: (1) Hyaluronic acid (HA) group: human adipose-derived stem cells were cultured in the hyaluronic acid/fibrin hydrogel but not treated with simvastatin (1 µM); and (2) Hyaluronic acid plus simvastatin (HA+ Sim) group: human adipose-derived stem cells were cultured in the hyaluronic acid/fibrin hydrogel and treated with simvastatin (1 µM). On days 7 and 14, hydrogels were collected and incubated in papain solution (300 µg/ml) for 18 hours at 60 °C. Then, 50 µl of the cell extract was obtained from each experimental group to detect the synthesis of sGAG by using the DMMB assay or to analyze type II collagen by using Type II Collagen Detection Kit (Chondrex Inc. WA, USA). The results were shown in Figures 4 and 5.

When coated with the three-dimensional hyaluronic acid hydrogel, whether 1 µM of simvastatin would effectively enhance the formation of two major extracellular matrix in mesenchymal stem cells, namely, sulfated glycosaminoglycans (sGAG) and type II collagen (Col II). As for the expression level of sGAG, on day 7, the hyaluronic acid (HA) group was 14 µg/µg, the hyaluronic acid plus simvastatin (HA+Sim) group was 18 µg/µg; on day 14, the hyaluronic acid group was 14 µg/µg, and the hyaluronic acid plus simvastatin group was 30 µg/µg (as shown in Figure 4). As for the expression level of Col II, on day 7, the hyaluronic acid (HA) group was 2 pg/µg, the hyaluronic plus simvastatin (HA+SIM) group was 6 pg/µg; on day 14, the hyaluronic acid group was 6.5 pg/µg and the hyaluronic acid plus simvastatin group was 27 pg/µg (as shown in Figure 5). The above results showed that when coated with the three-dimensional hyaluronic acid/fibrin hydrogel, 1 µM of simvastatin (HA+Sim) effectively enhanced the expression of two major extracellular matrix (sGAGs and Col II) in stem cells.

In addition, the present invention further utilized hyaluronic acid/fibrin hydrogel to culture human adipose-derived stem cells and treated them with or without 1 µM of simvastatin for 1 to 5 days to analyze the expression of chondrogenic genes (Sox-9, Aggrecan and type II collagen (Col II)). Therefore, in the present invention, there were two experimental groups: (1) Hyaluronic acid (HA) group: human adipose-derived stem cells were cultured in the hyaluronic acid/fibrin hydrogel but not treated with simvastatin (1 µM); and (2) hyaluronic acid plus simvastatin (HA+Sim) group: human adipose-derived stem cells were cultured in the hyaluronic acid/fibrin hydrogel and treated with simvastatin (1 µM). The cells were collected on designated days and the expression of chondrogenic genes was analyzed by using real-time PCR. The results are shown in Figure 6.

When coated with the three-dimensional hyaluronic acid hydrogel, whether 1 µM of simvastatin would effectively and preferably enhance the expression of chondrogenic genes in mesenchymal stem cells. As shown in Figures 6A to 6C, when stems cells were coated with the 3-dimensional hyaluronic acid/fibrin hydrogel, the effect of better expression of the chondrogenic genes was obtained when 1 µM of simvastatin (HA+Sim) was administered, that is, the expression of Sox-9, aggrecan and Col II was increased. This experiment showed that when coated with the three-dimensional hyaluronic acid hydrogel, the effect of better expression of the chondrogenic genes in stem cells could be obtained when 1 µM of simvastatin (HA+Sim) was administered.

### Example 4

40 mg of simvastatin (Merck & Company, Rahway, NJ, USA) was dissolved in 1 ml of ethanol and then mixed with 1.5 ml of 0.1 N NaOH. The simvastatin stock solution was heated to 50 °C for 2 hours and then the pH value was neutralized with 1 N of hydrochloric acid (HCl, pH = 7.4, Sigma-Aldrich, St Louis, MO, USA) to form a 10 mM of stock solution. Microspheres were then prepared by a water-in-oil-in-water (w/o/w) double emulsion technique. In short, 16 mg of hydroxyapatite (HAp) powder was dissolved in phosphate buffered saline (PBS) solution to form a first water-phase solution. 250 µl of simvastatin stock solution, 50 mg of surfactant Span 80 and polylactic-co-glycolic acid (PLGA) 50/50 (P2191, molecular weight: 30,000-60,000, Sigma-Aldrich) were mixed in dichloromethane solution to form an oil phase. The first water-phase solution and the oil phase solution were mixed with each other and stirred at 1000 rpm for 15 minutes to form a first water-in-oil (w/o) emulsion. The first water-in-oil (w/o) emulsion was added into 20 ml of the second water-phase solution and mixed with a 1% (w/v) of dichloromethane solution to form a second double emulsion (w/o/w). The second double emulsified (w/o/w) suspension was stirred at room temperature for 30 minutes to harden the microspheres. While stirring the suspension, exhaustion was essential to evaporate dichloromethane and to harden the microspheres. Finally, the microspheres were collected by centrifugation and washed three times with 0.1% of polyvinyl alcohol before being lyophilized in a lyophilizer.

All experimental animals were provided by Taitung Animal Propagation Station (TAPS). Animals were anesthetized by 0.15 mg/kg of Ketamine and sedative xylazine administered via intramuscular injection prior to surgery. During surgery, animals were given 5% of inhalational Isoflurane to maintain anesthesia. Therefore, after the miniature pigs had been anesthetized the operation was done on left legs. Full-thickness cartilage defects (6 mm in diameter) were created on the medial condyles of the left leg by using a trephine but which did not penetrate through the subchondral bone. After surgery, the hyaluronic acid/fibrin hydrogel containing 1 mg of simvastatin microspheres was implanted into the cartilage defect site which was covered with a periosteum obtained from the tibia for suturing and repairing the structure. Finally, the surgical wound site was stitched together layer by layer. After surgery, painkiller Ultracet (Tramadol 37.5 mg/Acetaminophen 325 mg) was given orally to the animals once daily, two tablets each time for 5 days. At week 12, the effect of treatment was monitored by observation.

As shown in Figure 7, when the three-dimensional hyaluronic acid hydrogel, which contained mesenchymal stem cells and simvastatin-containing microspheres, was administered to the articular cartilage defect site of the miniature pigs, it was found that after 12 weeks the articular cartilage defect site was significantly improved. The above results indicated that the mesenchymal stem cells coated with three-dimensional hyaluronic acid hydrogel and the simvastatin-containing microspheres had the effect of repairing and improving the articular cartilage defects.

## Claims

1. Pharmaceutical composition for use in promoting chondrogenesis, wherein the pharmaceutical composition comprises a hyaluronic acid mixture and a statin compound.

2. Pharmaceutical composition for use according to claim 1, wherein the hyaluronic acid mixture comprises hyaluronic acid, fibrin and a stem cell.

3. Pharmaceutical composition for use according to claim 2, wherein the fibrin forms a hydrogel.

4. Pharmaceutical composition for use according to claim 2, wherein the stem cell is human adipose-derived stem cell.

5. Pharmaceutical composition for use according to claim 1, wherein the statin compound is simvastatin.

6. Pharmaceutical composition for use according to claim 5, wherein the simvastatin is in the dosage form of a microsphere.

7. Pharmaceutical composition for use according to claim 1, wherein the pharmaceutical composition further inhibits osteosclerosis of the generated chondrocytes.

8. Pharmaceutical composition for use according to claim 7, wherein the pharmaceutical composition inhibits osteosclerosis of the generated chondrocytes by inhibiting the expression of osteocalcin.

9. Pharmaceutical composition for use according to claim 1, wherein chondrogenesis is promoted by increasing the expression of bone morphogenetic protein-2 (BMP-2).

10. Pharmaceutical composition for use according to claim 1, wherein chondrogenesis is promoted by increasing the expression of sulfated glycosaminoglycans (sGAG).

11. Pharmaceutical composition for use according to claim 1, wherein chondrogenesis is promoted by increasing the expression of chondrogenic genes comprising Sox-9, aggrecan or type II collagen.

12. Pharmaceutical composition for use according to claim 2, wherein the concentration of the hyaluronic acid in the pharmaceutical composition is 1 wt%.

13. Pharmaceutical composition for use according to claim 5, wherein the concentration of simvastatin in the pharmaceutical composition is 1 µM.

## Patentansprüche

1. Pharmazeutische Zusammensetzung für eine Verwendung bei einer Förderung einer Chondrogenese, wobei die pharmazeutische Zusammensetzung eine Hyaluronsäuremischung und eine Statinverbindung umfasst.

2. Pharmazeutische Zusammensetzung für die Verwendung nach Anspruch 1, wobei die Hyaluronsäuremischung Hyaluronsäure, Fibrin und eine Stammzelle umfasst.

3. Pharmazeutische Zusammensetzung für die Verwendung nach Anspruch 2, wobei das Fibrin ein Hydrogel ausbildet.

4. Pharmazeutische Zusammensetzung für die Verwendung nach Anspruch 2, wobei die Stammzelle eine von menschlichem Fettgewebe abstammende Stammzelle ist.

5. Pharmazeutische Zusammensetzung für die Verwendung nach Anspruch 1, wobei die Statinverbindung Simvastatin ist.

6. Pharmazeutische Zusammensetzung für die Verwendung nach Anspruch 5, wobei das Simvastatin in der Darreichungsform einer Mikrokugel ist.

7. Pharmazeutische Zusammensetzung für die Verwendung nach Anspruch 1, wobei die pharmazeutische Zusammensetzung ferner die Osteosklerose der erzeugten Chondrozyten hemmt.

8. Pharmazeutische Zusammensetzung für die Verwendung nach Anspruch 7, wobei die pharmazeutische Zusammensetzung die Osteosklerose der erzeugten Chondrozyten durch Hemmung der Expression eines Osteocalcins hemmt.

9. Pharmazeutische Zusammensetzung für die Verwendung nach Anspruch 1, wobei die Chondrogenese durch Erhöhung der Expression eines knochenmorphogenetischen Proteins-2 (*bone morphogenetic protein-2* - BMP-2) gefördert wird.

10. Pharmazeutische Zusammensetzung für die Verwendung nach Anspruch 1, wobei die Chondrogenese durch Erhöhung der Expression von sulfatierten Glykosaminoglykanen (sGAG) gefördert wird.

11. Pharmazeutische Zusammensetzung für die Verwendung nach Anspruch 1, wobei die Chondrogenese durch Erhöhung der Expression von chondrogenen Genen, umfassend Sox-9, Aggrecan oder Typ-II-Kollagen, gefördert wird.

12. Pharmazeutische Zusammensetzung für die Verwendung nach Anspruch 2, wobei die Konzentration der Hyaluronsäure in der pharmazeutischen Zusammensetzung 1 Gew.-% beträgt.

13. Pharmazeutische Zusammensetzung für die Verwendung nach Anspruch 5, wobei die Konzentration von Simvastatin in der pharmazeutischen Zusammensetzung 1 µM beträgt.

## Revendications

1. Composition pharmaceutique destinée à être utilisée pour favoriser la chondrogenèse, la composition pharmaceutique comprenant un mélange d'acide hyaluronique et un composé de statine.

2. Composition pharmaceutique destinée à être utilisée selon la revendication 1, dans laquelle le mélange d'acide hyaluronique comprend de l'acide hyaluronique, de la fibrine et une cellule souche.

3. Composition pharmaceutique destinée à être utilisée selon la revendication 2, dans laquelle la fibrine forme un hydrogel.

4. Composition pharmaceutique destinée à être utilisée selon la revendication 2, dans laquelle la cellule souche est une cellule souche dérivée d'adipose humaine.

5. Composition pharmaceutique destinée à être utilisée selon la revendication 1, dans laquelle le composé de statine est la simvastatine.

6. Composition pharmaceutique destinée à être utilisée selon la revendication 5, dans laquelle la simvastatine se présente sous la forme galénique d'une microsphère.

7. Composition pharmaceutique destinée à être utilisée selon la revendication 1, dans laquelle la composition pharmaceutique inhibe en outre l'ostéosclérose des chondrocytes générés.

8. Composition pharmaceutique destinée à être utilisée selon la revendication 7, dans laquelle la composition pharmaceutique inhibe l'ostéosclérose des chondrocytes générés en inhibant l'expression de l'ostéocalcine.

9. Composition pharmaceutique destinée à être utilisée selon la revendication 1, dans laquelle la chondrogénèse est favorisée en augmentant l'expression de la protéine morphogénétique osseuse-2 (BMP-2).

10. Composition pharmaceutique destinée à être utilisée selon la revendication 1, dans laquelle la chondrogénèse est favorisée en augmentant l'expression des glycosaminoglycanes sulfatés (GAG).

11. Composition pharmaceutique destinée à être utilisée selon la revendication 1, dans laquelle la chondrogénèse est favorisée en augmentant l'expression des gènes chondrogènes comprenant du Sox-9, de l'aggrécane ou du collagène de type II.

12. Composition pharmaceutique destinée à être utilisée selon la revendication 2, dans laquelle la concentration de l'acide hyaluronique dans la composition pharmaceutique est de 1 % en poids.

13. Composition pharmaceutique destinée à être utilisée selon la revendication 5, dans laquelle la concentration de simvastatine dans la composition pharmaceutique est de 1 µM.


### SEQUENCE LISTING

<110> KAOHSIUNG MEDICAL UNIVERSITY
<120> USE OF PHARMACEUTICAL COMPOSITION IN PREPARATION OF DRUG FOR PROMOTING CHONDROCYTE GENERATION
<130> 14197PCT
<160> 12
<170> PatentIn version 3.5
<210> 1
   <211> 20
   <212> DNA
   <213> Artificial Sequence
<220>
   <223> primer
<400> 1
   cgaatgactg gattgtggct 20
<210> 2
   <211> 20
   <212> DNA
   <213> Artificial Sequence
<220>
   <223> primer
<400> 2
   tgagttctgt cgggacacag 20
<210> 3
   <211> 18
   <212> DNA
   <213> Artificial Sequence
<220>
   <223> primer
<400> 3
   cttccgcgac gtggacat 18
<210> 4
   <211> 18
   <212> DNA
   <213> Artificial Sequence
<220>
   <223> primer
<400> 4
   gttgggcggc aggtactg 18
<210> 5
   <211> 21
   <212> DNA
   <213> Artificial Sequence
<220>
   <223> primer
<400> 5
   caacactgcc aacgtccaga t 21
<210> 6
   <211> 24
   <212> DNA
   <213> Artificial Sequence
<220>
   <223> primer
<400> 6
   tcttgcagtg gtaggtgatg ttct 24
<210> 7
   <211> 20
   <212> DNA
   <213> Artificial Sequence
<220>
   <223> primer
<400> 7
   acacgtgggg acattagtgg 20
<210> 8
   <211> 20
   <212> DNA
   <213> Artificial Sequence
<220>
   <223> primer
<400> 8
   gtggaatgca gaggtggttt 20
<210> 9
   <211> 23
   <212> DNA
   <213> Artificial Sequence
<220>
   <223> primer
<400> 9
   tctcctctgc attcaacagc gac 23
<210> 10
   <211> 21
   <212> DNA
   <213> Artificial Sequence
<220>
   <223> primer
<400> 10
   ccctgttgct gtagccaaat t 21
<210> 11
   <211> 20
   <212> DNA
   <213> Artificial Sequence
<220>
   <223> primer
<400> 11
   gtgcagagtc cagcaaaggt 20
<210> 12
   <211> 19
   <212> DNA
   <213> Artificial Sequence
<220>
   <223> primer
<400> 12
   cgataggcct cctgaaagc 19